# EUROPEAN PATENT APPLICATION

(11) **EP 4 775 956 A1**
(43) Date of publication of application: **15.07.2026**
(21) Application number: 26150345.2
(22) Date of filing: 06.01.2026
(51) Int. Cl.: G01N 21/03, G01N 21/05

(54) **THERMALLY COMPENSATED MULTI-PASS SPECTROSCOPY GAS CELL**

(30) Priority: 12.01.2025 US 202563744284 P; 12.12.2025 US 202519418497
(71) Applicant: Honeywell International Inc., Charlotte, NC 28202 (US)
(72) Inventor: FENG, Chen, Charlotte, 28202 (US); LEE, Seong Eyl, Charlotte, 28202 (US); SHAFAI, Moin S, Charlotte, 28202 (US)
(74) Representative: Haseltine Lake Kempner LLP

(57) **Abstract**

The present disclosure provides a thermally compensated multi-pass spectroscopy gas cell comprising a cylindrical cell housing constructed from a first material having a first coefficient of thermal expansion (CTE) and defining an interior. The cell comprises first and second mirror assemblies, each comprising a mirror holder and mirror secured within the holder. The mirror assemblies are positioned at opposite ends of the cylindrical cell housing to create a multi-pass optical configuration. The mirror holders are constructed from a second material having a second CTE that is greater than the first CTE to provide thermal compensation for stable mirror separation across operating temperature range.

## Description

### CROSS-REFERENCE TO RELATED APPLICATIONS

This application claims priority to U.S. Provisional Patent Application No. 63/744,284, filed on January 12, 2025, which is incorporated herein by reference in its entirety.

### TECHNICAL FIELD

Embodiments of the present disclosure generally relate to gas spectroscopy, and, more particularly, to thermally compensated multi-pass spectroscopy gas cells and systems.

### BACKGROUND

Applicant has identified many technical challenges associated with thermal stability in gas cells, including multi-pass spectroscopy gas cells. Through applied effort, ingenuity, and innovation, many of these identified problems have been solved by developing solutions that are included in embodiments of the present disclosure, many examples of which are described in detail herein.

### SUMMARY

Various embodiments of the present disclosure generally relate to gas spectroscopy, and, more particularly, to thermally compensated multi-pass spectroscopy gas cells.

According to an aspect of the present disclosure, a thermally compensated multi-pass spectroscopy gas cell is provided. The thermally compensated multi-pass spectroscopy gas cell comprises a cylindrical cell housing constructed from a first material having a first coefficient of thermal expansion (CTE) CTE_{cell_cyl} and defining an interior. The thermally compensated multi-pass spectroscopy gas cell comprises a first mirror assembly comprising a first mirror holder and a first mirror secured within the first mirror holder. The thermally compensated multi-pass spectroscopy gas cell comprises a second mirror assembly comprising a second mirror holder and a second mirror secured within the second mirror holder. The first mirror assembly and the second mirror assembly are positioned at opposite ends of the cylindrical cell housing to create a multi-pass optical configuration. The first mirror holder and the second mirror holder are constructed from a second material having a second CTE CTE_{mirror_hldr} that is greater than the first CTE to provide thermal compensation for stable mirror separation across operating temperature range.

According to other aspects of the present disclosure, the thermally compensated multi-pass spectroscopy gas cell may include one or more of the following features. The cylindrical cell housing may define a first length L_{cell_cyl} and each of the first mirror holder and the second mirror may define a second length L_{mirror_hldr}, wherein the first length and the second length may be selected based on a thermal compensation relationship L_{cell_cyl} × CTE_{cell_cyl} = 2 × L_{mirror_hldr} × CTE_{mirror_hldr}. The first material may comprise carbon steel. The first CTE may be in range of 11.2 to 12.0 × 10⁻⁶ per degree Celsius. The second material may comprise polyvinylidene fluoride (PVDF). The second CTE may be in a range of approximately 102 to 158 × 10⁻⁶ per degree Celsius. The thermally compensated multi-pass spectroscopy gas cell may further comprise an inner tube positioned within the interior of the cylindrical cell housing, wherein the inner tube may be constructed from the second material. The first mirror and the second mirror may be constructed from fused silica. The thermally compensated multi-pass spectroscopy gas cell may further comprise a first window assembly and a second window assembly positioned at the opposite ends of the cylindrical cell housing, wherein the first window assembly may comprise a first window holder and a light inlet window secured within the first window holder, and wherein the second window assembly may comprise a second window holder and a light outlet window secured within the second window holder. The first window holder and the second window holder may be constructed from the second material. The light inlet window and the light outlet window may be constructed from fused silica and may have a wedged geometry. The first mirror may define a light inlet through-hole and the second mirror may define a light outlet through-hole, wherein the light inlet through-hole and the light inlet window may be aligned, and wherein the light outlet through-hole and the light outlet window may be aligned. The thermally compensated multi-pass spectroscopy gas cell may further comprise a chamber defined at least in part by the cylindrical cell housing, the first mirror, and the second mirror. The thermally compensated multi-pass spectroscopy gas cell may further comprise a gas inlet port for introducing gas samples into the chamber and a gas outlet port for removing gas samples from the chamber. The thermally compensated multi-pass spectroscopy gas cell may further comprise a pressure-temperature sensor for monitoring operating condition. The operating temperature range may be -20°C to +60°C.

According to another aspect of the present disclosure, a gas detection system is provided. The gas detection system comprises a thermally compensated multi-pass spectroscopy gas cell comprising a cylindrical cell housing constructed from a first material having a first CTE and defining an interior. The thermally compensated multi-pass spectroscopy gas cell comprises a first mirror assembly comprising a first mirror holder and a first mirror secured within the first mirror holder. The thermally compensated multi-pass spectroscopy gas cell comprises a second mirror assembly comprising a second mirror holder and a second mirror secured within the second mirror holder. The first mirror assembly and the second mirror assembly are positioned at opposite ends of the cylindrical cell housing to create a multi-pass optical configuration. The first mirror holder and the second mirror holder are constructed from a second material having a second CTE that is greater than the first CTE to provide thermal compensation for stable mirror separation across operating temperature range. The gas detection system comprises a light emitter configured to emit light beam toward the thermally compensated multi-pass spectroscopy gas cell. The gas detection system comprises a light receiver configured to receive light exiting the compensated multi-pass spectroscopy gas cell.

According to other aspects of the present disclosure, the gas detection system may include one or more of the following features. The first material may comprise carbon steel and the second material may comprise PVDF. The gas detection system may further comprise an inner tube positioned within the interior of the cylindrical cell housing, wherein the inner tube may be constructed from the second material. The first mirror and the second mirror may be constructed from fused silica.

The above summary is provided merely for purposes of summarizing some example embodiments to provide a basic understanding of some aspects of the present disclosure. Accordingly, it will be appreciated that the above-described embodiments are merely examples and should not be construed to narrow the scope or spirit of the disclosure in any way. It will be appreciated that the scope of the present disclosure encompasses many potential embodiments in addition to those here summarized, some of which will be further described below.

### BRIEF DESCRIPTION OF THE FIGURES

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 illustrates an example perspective view of a thermally compensated multi-pass spectroscopy gas cell in accordance with at least some example embodiments of the present disclosure.
FIG. 2 illustrates a portion of an example thermally compensated multi-pass spectroscopy gas cell in accordance with at least some example embodiments of the present disclosure.
FIG. 3 illustrates an exploded view of an example thermally compensated multi-pass spectroscopy gas cell in accordance with at least some example embodiments of the present disclosure.
FIG. 4A illustrates a side view of an example thermally compensated multi-pass spectroscopy gas cell in accordance with at least some example embodiments of the present disclosure.
FIGS. 4B-C each illustrate an example mirror of an example thermally compensated multi-pass spectroscopy gas cell in accordance with at least some example embodiments of the present disclosure.
FIG. 5 illustrates a portion of an example thermally compensated multi-pass spectroscopy gas cell illustrating mirror separation thermal compensation in accordance with at least some example embodiments of the present disclosure.
FIG. 6A illustrates example material CTE vs. temperature plot in accordance with at least some example embodiments of the present disclosure.
FIG. 6B illustrates example compensated CTE vs. temperature plot in accordance with at least some example embodiments of the present disclosure.
FIG. 6C illustrates example mirror separation thermal shift plot in accordance with at least some example embodiments of the present disclosure.
FIG. 7A illustrates example light beam propagation and alignment in example thermally compensated multi-pass spectroscopy gas cell in accordance with at least some embodiments of the present disclosure.
FIG. 7B illustrates example beam footprints in accordance with at least some embodiments of the present disclosure.
FIG. 8 illustrates mirror separation thermal analysis in accordance with at least some embodiments of the present disclosure.
FIG. 9 is a block diagram of an example system for gas spectroscopy in accordance with example embodiments of the present disclosure.

### DETAILED DESCRIPTION

Some embodiments of the present disclosure will now be described more fully hereinafter with reference to the accompanying figures, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

The term "or" is used herein in both the alternative and conjunctive sense, unless otherwise indicated. The terms "illustrative" and "example" are used to be examples with no indication of quality level. Terms such as "computing," "determining," "generating," and/or similar words are used herein interchangeably to refer to the creation, modification, or identification of data. Further, "based on," "based on in part on," "based at least on," "based upon," and/or similar words are used herein interchangeably in an open-ended manner such that they do not indicate being based only on or based solely on the referenced element or elements unless so indicated.

As used herein, terms such as "front," "rear," "top," "bottom," "left," "right," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The phrases "in one example," "according to one example," "in some examples," and the like generally mean that the particular feature, structure, or characteristic following the phrase may be included in at least one example of the present disclosure and may be included in more than one example of the present disclosure (importantly, such phrases do not necessarily refer to the same example).

If the specification states a component or feature "may," "can," "could," "should," "would," "preferably," "possibly," "typically," "optionally," "for example," "as an example," "in some examples," "often," or "might" (or other such language) be included or have a characteristic, that specific component or feature is not required to be included or to have the characteristic. Such component or feature may be optionally included in some examples, or it may be excluded.

The word "example" or "exemplary" is used herein to mean "serving as an example, instance, or illustration." Any implementation described herein as "example" or "exemplary" is not necessarily to be construed as preferred or advantageous over other implementations.

The term "electrically connected," "electronically coupled," "electronically coupling," "electronically couple," "in communication with," "in electronic communication with," or "connected" in the present disclosure refers to two or more elements or components being connected through wired means and/or wireless means, such that signals, electrical voltage/current, data and/or information may be transmitted to and/or received from these elements or components.

The term "component" may refer to an article, a device, or an apparatus that may comprise one or more surfaces, portions, layers and/or elements. For example, an example component may comprise one or more substrates that may provide underlying layer(s) for the component and may comprise one or more elements that may form part of and/or are disposed on top of the substrate. the term "element" may refer to an article, a device, or an apparatus that may provide one or more functionalities.

### Overview

Multi-pass spectroscopy gas cells utilize multiple reflections between mirrors to achieve extended optical path length for absorptive spectroscopy gas sensing. High numbers of reflections may require high cell thermal stability to maintain reflection accuracy across varying temperature conditions. Traditional approaches for achieving thermal stability in multi-pass spectroscopy gas cells face significant limitations that restrict their practical applications.

Conventional aluminum-based multi-pass gas cells may operate within a narrow temperature range of only a few degrees Celsius to maintain the reflection accuracy needed for multi-pass optical systems. This limited temperature range may restrict aluminum-based cells to laboratory-only applications, preventing their use in outdoor or real-world environments where temperature variations are common.

Alternative approaches using extreme low thermal expansion materials such as nickel-iron alloys (Invar), lithium aluminum silicon oxide glass ceramics (Zerodur), or similar ultra-low expansion materials may achieve thermal stability but suffer from high material costs and machining expenses. The extreme cost of these materials and their associated manufacturing processes may significantly limit the commercial viability of multi-pass spectroscopy gas sensing products and applications.

Various embodiments of the present disclosure provide a thermally compensated multi-pass spectroscopy gas cell that overcomes the above noted technical challenges and difficulties associated with spectroscopy gas cells at least in part by utilizing a combination of materials (e.g., combination of CTE's thereof) to provide improved cell thermal stability, and thus, enhanced reflection accuracy in the gas cell.

Moreover, various embodiments are associated with low material cost and manufacturing cost (e.g., utilize low material cost and manufacturing cost), which expands the applications in which multi-pass spectroscopy gas sensing techniques may be utilized. Some gas cells, such as for example, aluminum-based multi-pass gas cell (e.g., a low-cost aluminum structured multi-pass gas cell) may be used, or otherwise work, only in a narrow operating temperature range in order to achieve and maintain the reflection accuracy (e.g., multi-reflection accuracy.) Such narrow operating temperature range may limit such gas cells to lab use. Example embodiments of the present disclosure provide a thermally compensated multi-pass spectroscopy gas cell with a larger operating temperature range (e.g., extends the operating range of multi-pass spectroscopy gas cells) that allows for real-world utilization of multi-pass spectroscopy gas cells (e.g., out-door real-world use beyond the lab).

The system may employ a combination of materials with different coefficients of thermal expansion (CTEs) in the gas cell cylinder and mirror holder components to create a compensated thermal response. In some examples, the thermally compensated gas cell may utilize a carbon steel cell cylinder combined with polyvinylidene fluoride (PVDF) mirror holders to achieve thermal compensation. The difference in CTEs between these materials, with PVDF having approximately ten times higher thermal expansion than carbon steel, may enable thermal compensation through geometric design where short, recessed high-CTE PVDF mirror holders work against a long, low-CTE carbon steel cell cylinder.

The non-linear temperature-dependent CTE characteristics of PVDF may provide additional advantages for extending the operational temperature range. The compensation between the carbon steel cell cylinder and PVDF mirror holders may exhibit non-monotonous residual thermal effects with non-zero combined effective CTE at different temperatures. This bi-directional combined effective CTE behavior may further extend the operation temperature range to cover real-world applications.

In this regard, various embodiments of the present disclosure provide a thermally compensated multi-pass spectroscopy gas cell addresses the above-noted limitations of existing system by utilizing a material combination approach that achieves thermal stability over a wide operating temperature range while maintaining low material and manufacturing costs.

### EXAMPLE SYSTEMS AND APPARATUSES OF THE DISCLOSURE

As noted above, various embodiments address technical problems associated with thermal stability in gas cells. Thermal stability may refer to the ability of a gas cell to maintain consistent optical and mechanical properties across varying temperature conditions, which enables accurate and reliable spectroscopic measurements.

Spectroscopy analysis of a sample (e.g., a fluid sample) may be used to determine one or more components within the fluid sample by illuminating the fluid sample with specific wavelengths of light and monitoring the absorption of light over an optical path length. Each component of the sample (atomic or molecular) may have a unique absorption signature that may be used to identify the presence and/or concentration of the component within the sample, or in some examples, the absence of a target gas (e.g., a leaked toxic gas) in the sample. For example, the concentration of methane, carbon dioxide, and/or other gases may be measured by illuminating the sample with varying wavelengths of light and analyzing the absorption signature across the various wavelengths. The accuracy of such measurements may depend on maintaining precise optical path lengths and mirror positions, which can be significantly affected by thermal expansion and contraction of the gas cell components.

A multi-pass spectroscopy gas cell may utilize multiple reflections between mirrors thereof to achieve extended optical path length for absorptive spectroscopy gas sensing. In such multi-pass configuration, light may bounce between opposing mirrors multiple times before exiting the cell, effectively increasing the interaction path length with the gas sample without requiring a physically longer cell. To achieve and maintain reflection accuracy, cell thermal stability (e.g., high thermal stability with respect to the gas cell) may be required. Even small changes in mirror separation due to thermal expansion can cause the light beam to deviate from its intended path, potentially resulting in beam clipping, reduced signal intensity, or complete loss of optical alignment.

Various embodiments of the present disclosure provide a thermally compensated spectroscopy gas cell with thermal compensation between components of the gas cell by utilizing a combination of material coefficients of thermal expansion (CTE's) to achieve and maintain the required thermal stability. In various embodiments, the coefficient of thermal expansion (CTE) is a property of a material that quantifies how much the material expands or contracts per unit length per degree of temperature change, and may be expressed in units of 1/°C or ppm/°C..

### Thermally compensated multi-pass spectroscopy gas cell

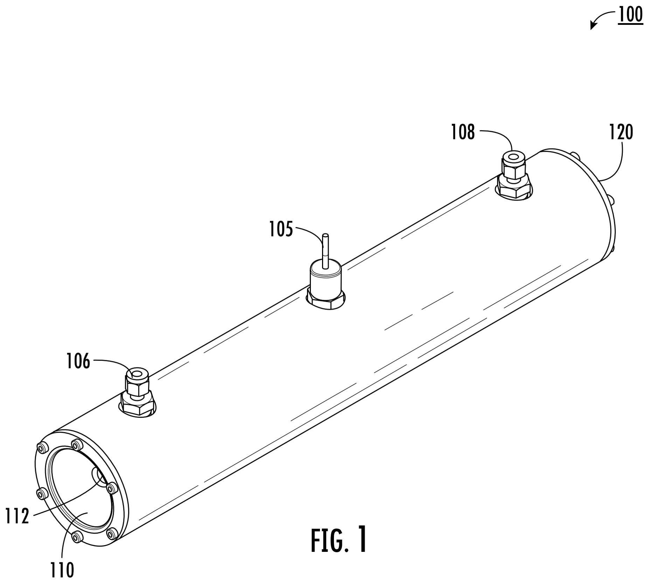

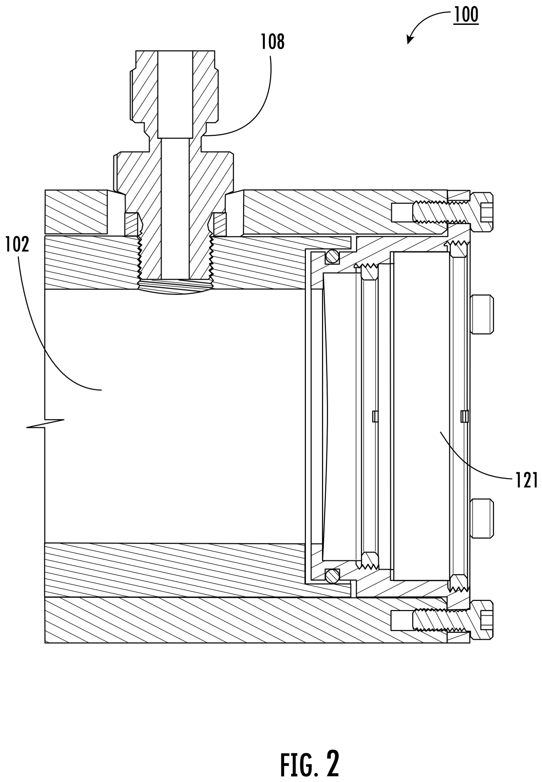

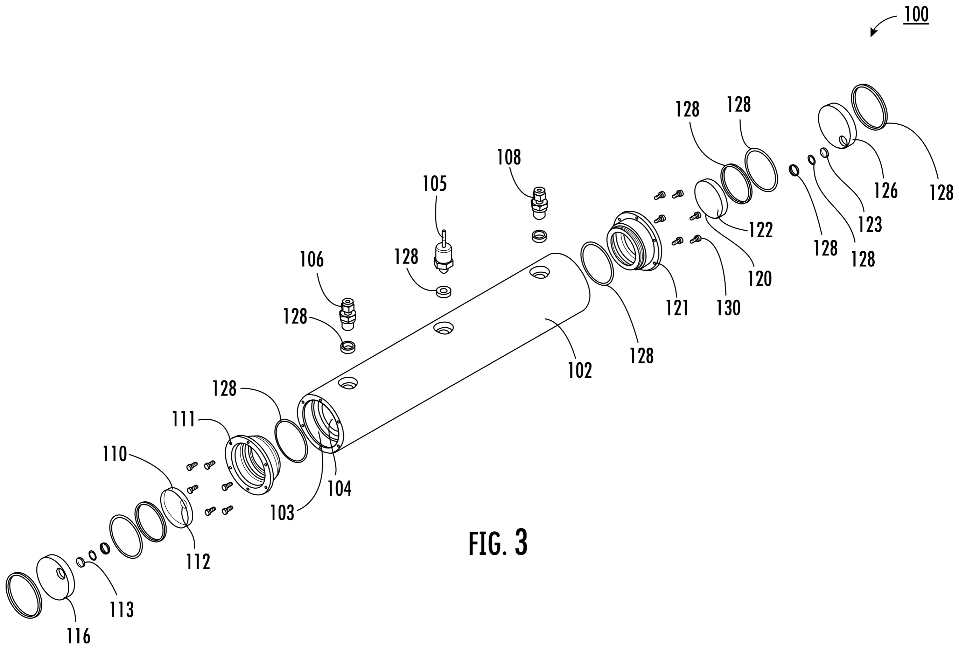

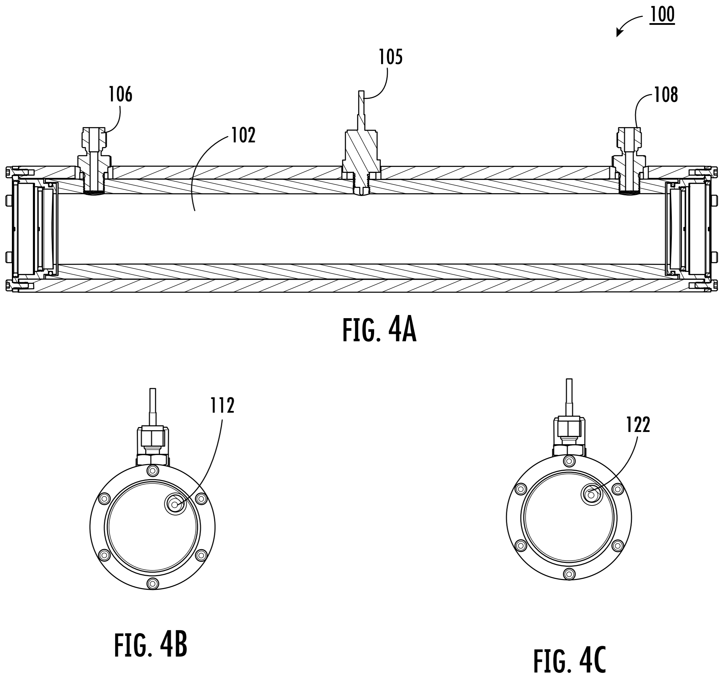

Now referring to FIGS. 1-4A, a thermally compensated multi-pass spectroscopy gas cell 100 is provided in accordance with at least some example embodiments of the present disclosure. Specifically, FIG. 1 illustrates a perspective view of an example thermally compensated multi-pass spectroscopy gas cell 100 in accordance with at least some example embodiments of the present disclosure. FIG. 2 illustrates a portion of the example thermally compensated multi-pass spectroscopy gas cell 100. FIG. 3 illustrates an exploded view of the example thermally compensated multi-pass spectroscopy gas cell 100. FIG. 4A illustrates a center cross sectional side view of the example thermally compensated multi-pass spectroscopy gas cell 100. The thermally compensated multi-pass spectroscopy gas cell 100 may comprise various components that work together to provide stable optical performance across a wide temperature range. The thermally compensated multi-pass spectroscopy gas cell 100 may include a gas cell cylinder, mirrors with associated mirror holders, optical window elements with window holders, gas flow components, and sensing elements.

In various embodiments, the example thermally compensated multi-pass spectroscopy gas cell 100 comprises a cell housing 102 defining an interior. In various embodiments, the cell housing 102 may have a cylindrical shape. For example, the cell housing 102 may comprise a cylindrical body. In this regard, in some embodiments the multi-pass spectroscopy gas cell 100 may comprise a gas cell cylinder (e.g., cylindrical cell housing 102). As shown in FIG. 3, in various embodiments, the thermally compensated multi-pass spectroscopy gas cell 100 may comprise an inner tube 103 positioned within the interior of the cell housing 102. The inner tube 103 may serve as an inner liner that provides chemical resistance and/or helps define the gas flow path within the cell housing 102.

In various embodiments, the thermally compensated multi-pass spectroscopy gas cell 100 comprises a first mirror 110 (also referred to herein as inlet mirror) and a second mirror 120 (also referred to herein as outlet mirror) affixed, or otherwise, coupled to opposing ends of the cell housing 102, thereby defining a chamber 104 (e.g., cavity or similar terms) as shown in FIG. 3. The chamber 104 may be configured for receiving a gas to be analyzed. The thermally compensated multi-pass spectroscopy gas cell 100 may comprise a first mirror holder 111 for receiving the first mirror 110 and a second mirror holder 121 for receiving the second mirror 120. The mirror holders (e.g., first mirror holder 111 and second mirror holder 121) may serve as mounting structures that position and secure the mirrors (e.g., mirror 110 and mirror 120). For example, the thermally compensated multi-pass spectroscopy gas cell 100 may include a pair of mirrors (e.g., first mirror 110 and second mirror 120) positioned within mirror holders (e.g., first mirror holder 111 and second mirror holder 121) to create multi-pass optical configuration. The first mirror 110 may be positioned within the first mirror holder 111 and the second mirror 120 may be positioned within the second mirror holder 121. In this regard, in various embodiments, the thermally compensated multi-pass spectroscopy gas cell 100 includes a first mirror assembly and a second mirror assembly positioned at opposite ends of the cell housing 102 to create a multi-pass optical configuration, where the first mirror assembly comprises the first mirror holder 111 and the first mirror 110 secured within the first mirror holder 111 and the second mirror assembly comprises the second mirror holder 121 and the second mirror 120 secured within the second mirror holder. The first mirror assembly and the second mirror assembly are positioned at opposite ends of the cylindrical cell housing to create a multi-pass optical configuration,

In various embodiments, the thermally compensated multi-pass spectroscopy gas cell 100 further comprises a pressure-temperature sensor 105 (e.g., combined pressure and temperature sensor), an inlet 106 via which the gas to be analyzed is added to the chamber 104 and an outlet 108 via which the gas to be analyzed is removed from the chamber 104. In this regard, the inlet 106 and outlet 108 may be configured to allow gas to flow through the gas cell for detection. In various embodiments, the pressure-temperature sensor 105 is configured to measure, detect, collect, and/or the like the gas flow pressure and temperature reading for gas sensing data analysis.

The cell housing 102 may form the primary structural framework of the thermally compensated multi-pass spectroscopy gas cell 100 and may define a length (e.g., overall length dimension) corresponding to the separation distance between the first mirror 110 and the second mirror 120. The cell housing 102 may be machined to precise dimensional tolerances to ensure proper optical alignment and may include mounting features for securing other components of the thermally compensated multi-pass spectroscopy gas cell 100 .

In various embodiments, the cell housing 102 is constructed from carbon steel material or other low thermal expansion material. The carbon steel configuration of the cell housing 102 may provide several advantages over other configurations, including aluminum. Carbon steel may provide a low coefficient of thermal expansion while maintaining cost-effectiveness compared to aluminum or some other low-expansion materials. For example, the cell housing 102 may be constructed from carbon steel having a CTE in the range of approximately 11.2 to 12.0 × 10⁻⁶ per degree Celsius, which represents a relatively lower CTE compared to many other structural materials (including aluminum) and is cost-effective relative to some other low-expansion materials such as Invar or Zerodur.

The relatively lower CTE exhibited by carbon steel may enable the cell housing 102 to serve as a stable structure against which higher expansion components may be positioned to achieve thermal compensation. The carbon steel cell housing 102 may define an interior (e.g., internal cavity) with precise dimensional tolerances that accommodate other components of the thermally compensated multi-pass spectroscopy gas cell 100 while maintaining structural stability across temperature variations. The material properties of carbon steel may provide adequate strength and rigidity to support the mirror assemblies and window assemblies while resisting deformation under thermal stress. Additionally, carbon steel may offer favorable machining characteristics that enable cost-effective manufacturing of the cell cylinder with the required dimensional accuracy. In this regard, the carbon steel construction of the cell housing 102 may provide structural stability and durability while maintaining manufacturability through machining processes.

An inner tube 103 may be positioned within the carbon steel cell cylinder to form or otherwise create a stable gas cell structure. In various embodiments, the inner tube is constructed from polyvinylidene fluoride (PVDF) or other high thermal expansion material. For example, PVDF may exhibit CTE in the range of approximately 102 to 158 × 10⁻⁶ per degree Celsius, which is higher (e.g., significantly higher) than the CTE of carbon steel from which the cell housing 102 may be constructed. The PVDF inner tube 103 may provide gas containment surface and chemical resistance to a wide range of gases. In other words, the PVDF inner tube 103 may provide a chemically inert surface that contacts the gas samples being analyzed. PVDF may offer excellent chemical resistance to a wide range of gases, which may prevent contamination or degradation of the thermally compensated multi-pass spectroscopy gas cell 100 over extended periods of operation.

The PVDF inner tube 103 may be dimensioned to fit (e.g., sliding fit) within the internal diameter of the carbon steel cell housing 102 while providing a defined gas volume for spectroscopic analysis. For example, the PVDF inner tube 103 may be dimensioned to fit (e.g., sliding fit) within the internal cavity of the carbon steel cell housing 102 while allowing for differential thermal expansion between the two materials. The inner tube 103 may be secured within the cylinder via sliding fit. The inner tube 103 may provide a smooth internal surface that minimizes gas flow turbulence and may facilitate cleaning or maintenance procedures.

The combination of the carbon steel cell housing 102 and the PVDF inner tube 103 may create a dual-material structure that balances thermal, chemical, and mechanical performance requirements. The carbon steel construction of the cell housing 102 may provide structural integrity and dimensional stability and may contribute to the thermal compensation scheme through its lower coefficient of thermal expansion, while the PVDF construction of the inner tube 1-3 may provide chemical compatibility. In this regard, the combination of the carbon steel cell housing 102 and the PVDF inner tube 103 may create a dual-wall structure that combines the structural benefits and lower thermal expansion characteristics of carbon steel with the chemical compatibility. Moreover, this material combination (e.g., carbon steel material of the cell housing 102 and PVDF material of the inner tube 103) may enable cost-effective manufacturing while achieving performance characteristics suitable for demanding gas sensing applications.

The material selection of carbon steel for the cell housing 102 and PVDF for the inner tube 103 may provide cost advantages compared to alternative approaches using exotic low-expansion materials. The cost-effectiveness of these materials (e.g., carbon steel and PVDF) may enable mass production of thermally compensated gas cells for widespread commercial applications where cost constraints may otherwise limit the adoption of multi-pass spectroscopy technology. Both carbon steel and PVDF may be readily available commercial materials that can be processed using standard manufacturing techniques.

The mirrors 110, 112 may provide optical performance characteristics for effective multi-pass spectroscopy. For example, the mirrors 110, 120 may be fused silica mirrors, wherein fused silica may provide high optical quality, low absorption, and/or excellent surface properties that enable efficient light reflection across multiple passes through the thermally compensated multi-pass spectroscopy gas cell 100 . The material properties of fused silica may also contribute to long-term stability and resistance to optical degradation under various operating conditions.

The mirror holders 111, 121 may be configured to securely host the mirrors 110, 120 while facilitating or otherwise providing thermal compensation as described herein. The mirror holders 111, 121 may include mounting features such as recesses, grooves, or mechanical retention elements that position and secure the mirrors in precise optical alignment.

In various the first mirror holder 111 and second mirror holder 121 are constructed from PVDF material, which may provide a high coefficient of thermal expansion that enables thermal compensation within the gas cell 100. As described above, PVDF material may exhibit a coefficient of thermal expansion in the range of approximately 102 to 158 × 10⁻⁶ per degree Celsius, which may be approximately ten times higher than the thermal expansion coefficient of the carbon steel material of the cell housing 102. This significant difference in thermal expansion properties may enable the mirror holders 111, 121 to provide compensating dimensional changes that counteract thermal expansion of the cell housing 102.

The geometry of the PVDF mirror holders 111, 121 may be specifically designed to utilize the high thermal expansion characteristics of PVDF for compensation purposes. For example, the PVDF mirror holders 111, 121 may be configured with specific geometric dimensions to achieve thermal compensation against the carbon steel cell housing 102. In some examples, the mirror holders 111, 121 may be configured as short, recessed components that extend into the cell housing 102 from each end of the cell housing 102 and that position the pair of mirrors 110, 120 at precise locations within the cell housing 102. In this regard, the higher coefficient of thermal expansion of PVDF may enable the use of relatively short mirror holder lengths to achieve the desired thermal compensation effect. This geometric advantage may simplify the mechanical design of the mirror assemblies while maintaining the structural integrity to support the mirrors in precise alignment. The PVDF material may also provide additional benefits including low material cost, favorable machining characteristics, and excellent chemical resistance that makes the material suitable for gas cell applications.

The geometric configuration may position the mirror holders 111, 121 such that their thermal expansion works in opposition to the thermal expansion of the longer carbon steel cell housing 102, thereby maintaining stable mirror separation distances across temperature variations. For example, as temperature increases, the high thermal expansion of the PVDF mirror holders 111, 121 may cause the mirror holders 111, 121 to expand and move the mirrors 110, 120 in a direction that counteracts the thermal expansion of the carbon steel cell housing 102.

The thermal compensation may operate through the differential expansion between the carbon steel cell housing 102 and the PVDF mirror holders 111, 121. For example, when temperature increases, the carbon steel cell housing 102 may expand and increase the overall internal length of the cell housing 102 or gas cell 100. Simultaneously, the PVDF mirror holders 111, 121 may expand at a higher rate due to their significantly higher coefficient of thermal expansion. The expansion of the PVDF mirror holders 111, 121 may move the mirrors inward relative to the ends of the cell housing 102, thereby reducing the effective mirror separation distance. In this regard, the thermal compensation may operate on the principle that as temperature increases, the cell housing 102 expands lengthwise, which would normally increase the mirror separation distance. However, the higher CTE mirror holders 111, 121 expand more rapidly, effectively pushing the mirrors 110, 120 inward to counteract the expansion of the cell housing 102. In this regard, in various embodiments, the low thermal carbon steel material of the cell housing 102 and the high thermal expansion PVDF of the mirror holders 111, 121 are selected to compensate the thermal change of the cell length with cancellation between cell housing 102 and the mirror holders 111, 121, and minimize the thermal position shift of the mirrors 110, 120.

The positioning and dimensioning of the PVDF mirror holders 111, 121 may be configured to achieve substantial cancellation of thermal effects across the operating temperature range. The relationship between the cell housing length, mirror holder length, and their respective coefficients of thermal expansion may determine the degree of thermal compensation achieved. In some examples, the thermal compensation may be designed such that the thermal expansion of the cell housing length multiplied by the cell cylinder CTE approximately equals twice the thermal expansion of the mirror holder length multiplied by the mirror holder CTE.

In some examples, the mirror separation within the thermally compensated multi-pass spectroscopy gas cell 100 may have a nominal length of 385.2 mm between the opposing mirrors 110, 120. This separation distance may be determined by the length of the cell housing 102 minus the combined lengths of the two mirror holders 111, 121 extending into the cylinder from each end. The 385.2 mm nominal separation may provide sufficient optical path length for multi-pass spectroscopic measurements while maintaining manageable overall assembly dimensions.

In some examples, the thermal compensation design may accommodate acceptable separation variations of approximately ±0.1 mm while maintaining proper optical performance. Within this tolerance range, the thermally compensated multi-pass spectroscopy gas cell 100 may maintain reflection accuracy and optical path stability across the intended operating temperature range. The ±0.1 mm variation tolerance may be achieved through careful selection of the length ratios between the carbon steel cell housing 102 and the PVDF mirror holders 111, 121 based on their respective thermal expansion coefficients.

The PVDF material of the mirror holders 111, 121 may provide sufficient structural stability to maintain mirror positioning while accommodating thermal expansion and contraction cycles without degrading optical performance. The combination of fused silica mirrors and PVDF mirror holders 111, 121 may create a mirror assembly that maintains stable optical performance while utilizing cost-effective materials. The thermal compensation provided by the PVDF mirror holders may enable the gas cell 102 to operate across extended temperature ranges without the optical losses or alignment degradation that may occur in non-compensated configurations or other configurations. This approach may eliminate the need for expensive ultra-low expansion materials while achieving comparable thermal stability performance. The mirror holders 111, 121 may also provide mounting interfaces for additional optical components. For example, the PVDF material properties may enable integration of window holders or other optical mounting features into the mirror holders 111, 121.

The thermally compensated multi-pass spectroscopy gas cell 100 may include optical assemblies that provide optical access for light introduction and collection while maintaining gas containment within the cell structure. In various embodiments, the thermally compensated multi-pass spectroscopy gas cell 100 comprises an inlet optical window element 113 (e.g., also referred to herein as light inlet window 113) and an outlet optical window element 123 (e.g., also referred to herein as light outlet window 123). In various embodiments, the first mirror 110 may define and/or comprise a light inlet through-hole 112 and the second mirror 120 may define and/or comprise a light outlet through-hole 122. The light inlet through-holes may be configured to allow light to enter and exit the chamber 104 (e.g., multi-pass cavity) while maintaining reflective surfaces for the multi-pass optical configuration.

In various embodiments, the optical window elements are positioned within the thermally compensated multi-pass spectroscopy gas cell 100 such that the inlet optical window element 113 aligns with the light inlet through-hole 112 and the outlet optical window element 123 aligns with the light outlet through-hole 122. In various embodiments, the inlet optical window element 113 is secured to the cell housing 102 via a first window holder 116 and the outlet optical window element 123 is secured to the cell housing 102 via a second window holder 126. In various embodiments, light beam enters the chamber 104 via the light inlet through-hole 112 and/or the inlet optical window element 113 and exits the chamber 104 via the light outlet through-hole 122 and/or the outlet optical window element 123. In some examples, the light inlet through-hole 112 and the light outlet through-hole 122 may be conical or other suitable shape to, for example, minimize unwanted reflections and/or optimize beam coupling.

As described above, the mirrors 110, 120 may be constructed of fused silica or other similar near-infrared optical material with high reflective coating that provides high reflectivity and low absorption in the spectroscopic wavelength range of interest. In various embodiments, the optical window elements 113, 123 are constructed from fused silica material or other similar near-infrared optical material with anti-reflective and may be configured with a wedged geometry to, for example, provide optical coupling to the thermally compensated multi-pass spectroscopy gas cell 100. For example, the thermally compensated multi-pass spectroscopy gas cell 100 may comprise a pair of fused silica wedged optical window elements 113, 123 (e.g., two fused silica wedged optical window elements 113, 123) where the wedge angle may be configured to, for example, reduce back-reflections that could interfere with the measurement.

The wedged geometry of the optical window elements 113, 123 may serve to minimize unwanted reflections that could interfere with the multi-pass optical configuration. When light encounters a parallel-sided optical window, a portion of the light may reflect from both the entrance and exit surfaces of the window. These reflections may create ghost images or spurious optical signals that can degrade the performance of the spectroscopic measurement. The wedged configuration may address this issue by angling one surface of the window relative to the other surface. The wedged window design may cause reflections from the entrance and exit surfaces to propagate in different directions rather than retracing the incident optical path. This angular separation of reflected beams may prevent the reflected light from interfering with the primary optical signal traveling through the thermally compensated multi-pass spectroscopy gas cell 100. The wedge angle may be selected to provide adequate separation of unwanted reflections while maintaining acceptable optical transmission through the window.

In various embodiments, the first window holder 116 and the second window holder 126 are constructed of PVDF. The PVDF window holders 116, 126 may secure and position the optical window elements 113, 123 within the thermally compensated multi-pass spectroscopy gas cell 100 while contributing to the overall thermal compensation. Similar to the PVDF mirror holders 111, 121, the PVDF window holders 116, 126 may exhibit the high coefficient of thermal expansion characteristic of PVDF material, which may range from approximately 102 to 158 × 10⁻⁶ per degree Celsius. This high thermal expansion rate may enable the window holders to expand and contract in coordination with the PVDF mirror holders 111, 121 as temperature varies.

The integration of the PVDF window holders 116, 126 with the PVDF mirror holders 111, 121 may create a coordinated thermal compensation system where the optical elements move together in response to temperature changes. This coordinated movement may maintain consistent optical alignment between the mirrors 110, 120 and optical window elements 113, 123 across the operating temperature range. The window holders 116, 126 may be positioned and dimensioned to ensure that the wedged optical window elements 113, 123 remain properly aligned with the optical path as the mirror positions adjust due to thermal compensation effects.

The PVDF window holders 116, 126 may provide additional benefits beyond thermal compensation, including chemical resistance that protects the window mounting interfaces from corrosive gases or vapors that may be present in the thermally compensated multi-pass spectroscopy gas cell 100. The material properties of PVDF may enable the window holders 116, 126 to maintain structural integrity and sealing performance across the full operating temperature range while accommodating the differential thermal expansion between the various cell components.

The window holders 116, 126 may be configured to accommodate the wedged geometry of the fused silica optical window elements 113, 123 while providing proper sealing and mechanical retention. The window holders 116, 126 may include mounting features such as recesses or grooves that conform to the wedged window element shapes and may provide secure positioning without inducing mechanical stress that could affect optical performance. The PVDF material may offer sufficient flexibility to accommodate thermal expansion differences between the holder material and the fused silica windows. In various embodiments, the first window holder 116 (e.g., inlet window holder) and the second window holder 126 (e.g., outlet window holder) may be housed within the first mirror holder 111 and the second mirror holder 121, respectively. The combination of wedged fused silica windows and PVDF window holders may enable effective optical performance while contributing to the overall cost-effectiveness and thermal stability of the gas cell system. Sealing elements may be incorporated between the window holders 116, 126 and the cell structure to prevent gas leakage while allowing for thermal movement.

The thermally compensated multi-pass spectroscopy gas cell 100 may incorporate seal rings, such as seal rings 128, to provide gas-tight integrity across various component interfaces while accommodating thermal expansion of the various materials. The seal rings may be positioned at strategic locations where gas containment may be compromised, such as between the mirror holders and the cell housing 102, between the window holders and the cell structure, and/or at other component interfaces where gas leakage could occur. These sealing elements may be constructed from materials that maintain sealing effectiveness across the full operating temperature range of the gas cell. In some embodiments, the seal rings may be fabricated from elastomeric materials that provide adequate compression and recovery characteristics to maintain sealing contact as the various cell components expand and contract with temperature changes. In some examples, the seal rings may comprise O-rings or other sealing elements that offer predictable sealing performance and cost-effective replacement when maintenance is required. The material selection for the seal rings may consider chemical compatibility with the gas samples being analyzed to prevent degradation or contamination of the sealing surfaces.

The thermally compensated multi-pass spectroscopy gas cell 100 may incorporate fasteners, such as fastener 130 to secure the various components of the gas cell 102 together while allowing for the thermal movement necessary for the compensation mechanism to function effectively. The fasteners may include bolts, screws, or other mechanical fastening elements that provide adequate clamping force to maintain structural integrity and sealing compression. The fastener materials and configurations may be selected to withstand the mechanical stresses associated with thermal cycling across the operating temperature range. The combination of seal rings and fasteners may create a complete sealing system that maintains gas-tight integrity throughout the thermal compensation process. As the PVDF components expand and contract relative to the carbon steel cell cylinder, the sealing components may accommodate these movements while preventing gas leakage that could affect measurement accuracy or create safety concerns.

In various embodiments, the thermally compensated multi-pass spectroscopy gas cell 100 includes a gas inlet port 106 and a gas outlet port 108. For example, a pair of gas inlet and outlet ports may be integrated into the gas cell structure to enable gas sample introduction and removal during spectroscopic analysis operations. The gas inlet and outlet ports 106, 108 may provide the flow path for introducing gas samples into the chamber 104 (e.g., cell cavity) and removing the samples after analysis is complete. The gas inlet and outlet configuration may enable continuous flow operation where fresh gas samples are continuously introduced while analyzed samples are removed, or batch operation where discrete gas samples are introduced, analyzed, and then removed.

The gas inlet port 106 may be positioned to introduce gas samples into the chamber 104 in a manner that promotes efficient mixing and uniform distribution throughout the optical path region. The gas inlet port 106 may be configured to minimize turbulence or flow disturbances that could affect the optical measurements by creating density variations or pressure fluctuations within the gas sample. The gas inlet port 106 may include appropriate fittings or connections that enable integration with external gas supply systems, sample handling equipment, or gas delivery manifolds. The gas outlet port 108 may be positioned to enable efficient removal of gas samples from the chamber 104 after spectroscopic analysis. The location of the gas outlet port 108 may be selected to facilitate complete gas exchange and prevent stagnant regions where old gas samples could remain and interfere with subsequent measurements. The gas outlet port 108 may also include appropriate fittings or connections for integration with external gas handling systems, vacuum pumps, or gas disposal systems depending on the specific application requirements. The positioning of the gas inlet and outlet ports 106, 108 within the gas cell structure may be configured to minimize interference with the multi-pass optical path while ensuring adequate gas flow characteristics. The gas inlet and outlet ports 106, 108 may be located away from the primary optical beam paths to prevent obstruction of the light reflections between the mirrors.

The thermally compensated multi-pass spectroscopy gas cell 100 may implement a gas flow system designed to accommodate various gas sample types and flow rates depending on the specific spectroscopic application requirements. The dimensions and configurations of the gas inlet and outlet ports 106, 108 may be selected to provide adequate flow capacity for the intended gas samples while maintaining appropriate residence times within the thermally compensated multi-pass spectroscopy gas cell 100 for effective optical absorption measurements. The gas inlet and outlet ports 106, 108 may be configured to handle different gas pressures, from subatmospheric conditions for vacuum applications to elevated pressures for high-pressure gas analysis.

The gas flow configuration enabled by the gas inlet and outlet ports 106, 108 may support various operational modes including continuous monitoring applications and batch sampling procedures. In continuous monitoring mode, gas may flow continuously through the cell, enabling real-time analysis of changing gas compositions. In batch sampling mode, discrete gas samples may be introduced into the cell for analysis, with the outlet port enabling removal of the sample after measurement completion. The flow configuration may accommodate different flow rates and pressure conditions depending on the specific application requirements.

The gas inlet and outlet ports 106, 108 may incorporate sealing elements to maintain gas-tight integrity at the port connections while allowing for thermal expansion of the cell structure. The sealing elements may prevent gas leakage that could affect measurement accuracy or create safety concerns, particularly when analyzing hazardous or toxic gas samples. The sealing approach at the gas ports may be coordinated with the overall sealing system of the gas cell to ensure consistent performance across the full operating temperature range. The gas flow path through the thermally compensated multi-pass spectroscopy gas cell 100 may be designed to provide uniform gas distribution across the optical path length while minimizing dead volumes where gas samples could stagnate. The internal flow configuration may promote laminar flow conditions that reduce optical disturbances while ensuring complete gas exchange during sample changeover operations. The flow path design may also consider the chemical compatibility requirements for the gas samples being analyzed to prevent contamination or degradation of the gas flow surfaces.

In various embodiments, the thermally compensated multi-pass spectroscopy gas cell 100 includes a combined pressure-temperature sensor 105. The pressure-temperature sensor 105 may be configured for monitoring the operating conditions within the gas sample during spectroscopic analysis operations. The pressure-temperature sensor 105 may provide simultaneous measurement of both gas pressure and temperature parameters, which may be utilized for comprehensive gas sensing data analysis and system monitoring. The combined sensor approach may offer advantages over separate pressure and temperature sensors by reducing the number of sensor penetrations into the gas cell while providing coordinated measurements from a single sensing location.

The pressure sensing capability of the pressure-temperature sensor 105 may measure the gas pressure within the cell cavity across a range of operating pressures suitable for various spectroscopic applications. The pressure measurements may be utilized to characterize the gas sample conditions and may enable compensation for pressure-dependent effects in the spectroscopic analysis. Gas absorption characteristics may vary with pressure due to pressure broadening effects, collision-induced absorption changes, and density variations that affect the optical path length effectiveness. The temperature sensing capability of the pressure-temperature sensor 105 may monitor the gas temperature within the cell cavity to provide data for temperature-dependent spectroscopic analysis corrections. Gas absorption spectra may exhibit temperature-dependent characteristics including line strength variations, spectral line broadening effects, and wavelength shifts that may affect measurement accuracy. The temperature measurements may enable real-time compensation for these temperature-dependent effects to improve the precision and reliability of the gas concentration determinations.

The combined pressure-temperature sensor 105 may provide measurement data that characterizes the thermodynamic state of the gas sample within the cell cavity. This state information may be utilized in gas sensing algorithms that account for the relationship between gas density, pressure, and temperature according to gas law relationships. The sensor data may enable conversion between different concentration units, such as parts per million by volume, mass concentration, or molar concentration, depending on the specific application requirements. The sensor measurements may be integrated with the spectroscopic data analysis to provide enhanced measurement accuracy and reliability. The pressure and temperature data may be utilized to normalize the spectroscopic absorption measurements for variations in gas density and molecular absorption characteristics. This normalization process may enable consistent gas concentration measurements across varying environmental conditions and may improve the long-term stability and repeatability of the spectroscopic analysis system.

The pressure-temperature sensor 105 may be positioned within the gas cell structure to provide representative measurements of the gas conditions in the optical path region where the spectroscopic analysis occurs. The location of the pressure-temperature sensor 105 may be selected to minimize interference with the multi-pass optical configuration while ensuring that the measured pressure and temperature values accurately reflect the conditions experienced by the gas sample during optical analysis. The sensor positioning may also consider the gas flow patterns within the cell to ensure that the measurements represent the bulk gas conditions rather than localized variations.

The pressure-temperature sensor 105 may be configured to operate across the full temperature range of the thermally compensated gas cell system (e.g., from approximately -20°C to +60°C) while maintaining measurement accuracy and reliability. The pressure-temperature sensor 105 may be constructed from materials and components that provide stable performance characteristics across this extended temperature range. The sensor calibration and compensation algorithms may account for temperature-dependent sensor characteristics to ensure accurate measurements throughout the operating temperature range.

The output signals of the pressure-temperature sensor 105 may be processed and transmitted to external data acquisition systems or gas analysis equipment for integration with the spectroscopic measurement data. The pressure-temperature sensor 105 may provide analog or digital output signals depending on the specific sensor design and system integration requirements. The sensor data may be synchronized with the optical measurements to enable real-time correlation between the gas conditions and the spectroscopic absorption characteristics.

The combined pressure-temperature sensor 105 may contribute to the overall cost-effectiveness and simplicity of the gas cell system by eliminating the need for separate pressure and temperature sensing devices. This integrated approach may reduce the complexity of the gas cell design while providing comprehensive monitoring of the gas sample conditions. The pressure-temperature sensor 105 may also provide diagnostic information about the gas cell operation, including detection of gas leaks, flow blockages, or other operational issues that could affect measurement performance.

### Thermal Compensation Mechanism

The thermal compensation mechanism may operate through the differential thermal expansion between the carbon steel cell cylinder and the PVDF mirror holders to maintain stable mirror separation across temperature variations. The compensation principle may be based on the substantial difference in coefficients of thermal expansion between these materials, where the PVDF material exhibits a coefficient of thermal expansion approximately 10 times greater than that of carbon steel. This differential may enable the design of a compensation system where thermal expansion effects substantially cancel each other out.

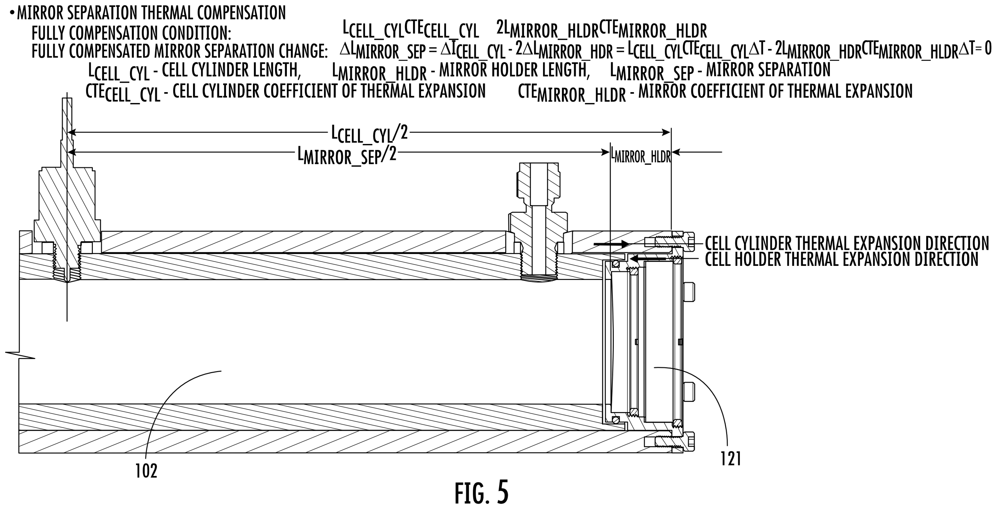

As shown in FIG. 5, the full compensation condition may be expressed as L_{cell_cyl} × CTE_{cell_cyl} = 2 × L_{mirror_hldr} × CTE_{mirror_hldr}, where L_{cell_cyl} represents the cell cylinder length, CTE_{cell_cyl} represents the coefficient of thermal expansion of the cell cylinder material, L_{mirror_hldr} represents the mirror holder length, and CTE_{mirror_hldr} represents the coefficient of thermal expansion of the mirror holder material. This relationship may define the geometric proportions necessary to achieve thermal compensation between the expanding cell cylinder and the expanding mirror holders.

In this regard, in some embodiments, one or more components of the thermally compensated multi-pass spectroscopy gas cell 100 may be selected based on the full compensation condition (e.g., thermal compensation relationship). For example, the length of the cell housing 102 and/or length of the mirror holders 111, 121 may be selected based on the thermal compensation condition.

The compensated mirror separation change may be express as or calculated using the equation ΔL_{mirror_sep} = L_{cell_cyl} × CTE_{cell_cyl} × ΔT - 2 × L_{mirror_hdr} × CTE_{mirror_hldr} × ΔT = 0, where ΔL_{mirror_sep} represents the change in mirror separation, ΔT represents the temperature change, and the factor of 2 accounts for the presence of mirror holders at both ends of the cell cylinder. When this equation equals zero, the thermal expansion of the cell cylinder may be balanced by the thermal expansion of the mirror holders, resulting in no net change in mirror separation.

The carbon steel cell cylinder may exhibit a coefficient of thermal expansion in the range of 11.2 to 12.0 × 10⁻⁶ per degree Celsius providing a relatively low thermal expansion rate that serves as the baseline for the compensation mechanism. The PVDF mirror holders may exhibit a coefficient of thermal expansion in the range of 102 to 158 × 10⁻⁶ per degree Celsius, which represents approximately 8 to 14 times the expansion rate of the carbon steel material. This difference in thermal expansion coefficients may enable the use of geometrically shorter recessed PVDF mirror holders to counteract the thermal expansion of the longer carbon steel cell cylinder.

The geometric advantage provided by the differential thermal expansion rates may allow the mirror holders to be designed as relatively short components while still providing adequate compensation for the thermal expansion of the much longer cell cylinder. The high coefficient of thermal expansion of the PVDF material may enable a small mirror holder length created change to produce a compensation effect equivalent to a much larger cell cylinder length created change. This geometric relationship may simplify the mechanical design while maintaining the structural integrity necessary for precise mirror positioning.

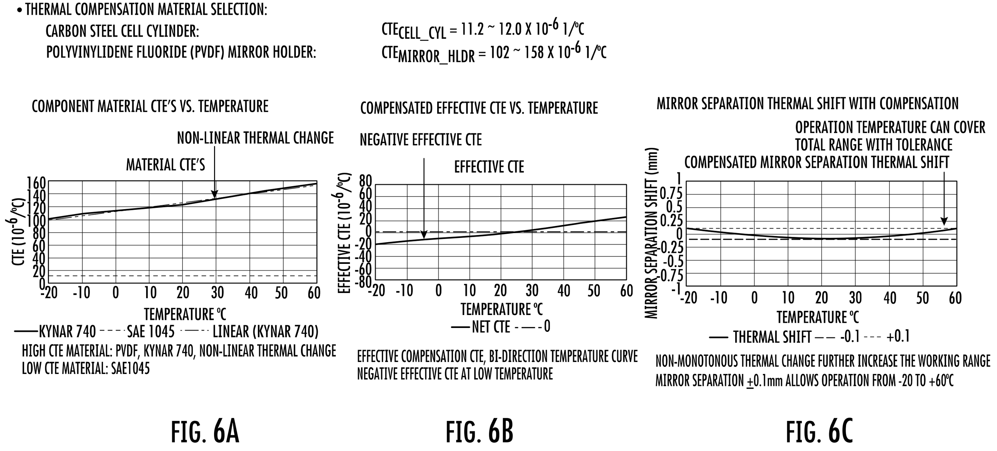

Now referencing FIGS. 6A-C, the thermal compensation mechanism may achieve mirror separation variation tolerance of ±0.1 mm over the operating temperature range through careful selection of the material coefficients and geometric proportions. This tolerance level may be sufficient to maintain the optical alignment accuracy necessary for effective multi-pass spectroscopy operation while accommodating the thermal expansion effects that occur across the extended temperature range from approximately -20°C to +60°C.

As shown in FIG. 6A, the PVDF material may exhibit non-linear temperature dependent coefficient of thermal expansion characteristics. This non-linear behavior may result in non-monotonous compensation characteristics where the combined effective coefficient of thermal expansion varies across the temperature range.

As shown in FIG. 6B, the non-linear thermal expansion behavior of PVDF may create a compensation system with negative combined effective coefficient of thermal expansion at low temperatures and positive combined effective coefficient of thermal expansion at high temperatures. At low temperatures, the thermal compensation may result in a net contraction of the mirror separation as temperature increases, while at high temperatures, the compensation may result in a net expansion of the mirror separation as temperature increases. This bi-directional compensation behavior may extend the effective operating temperature range by providing compensation in both directions from a central temperature point.

As shown in FIG. 6C, the non-monotonous compensation behavior may enable the gas cell system to maintain mirror separation within the required tolerance across a broader temperature range than would be possible with linear thermal expansion materials. The varying compensation characteristics may provide enhanced thermal stability at temperature extremes where conventional linear compensation approaches might exceed acceptable tolerance limits. This extended temperature range capability may enable the gas cell to operate effectively in real-world applications where temperature variations are substantial and unpredictable.

The thermal compensation mechanism may function automatically without requiring active control or adjustment systems. As temperature changes occur, the differential thermal expansion between the carbon steel cell cylinder and the PVDF mirror holders may immediately respond to maintain stable mirror positioning. This passive compensation approach may provide reliable thermal stability without the complexity, cost, and potential failure modes associated with active temperature control systems.

### System Operation

During operation, the thermally compensated multi-pass spectroscopy gas cell 100 may function as an integrated system where optical, thermal, and gas flow components work together to enable accurate spectroscopic measurements across a wide temperature range. For example, the thermally compensated multi-pass spectroscopy gas cell 100 may implement a multi-pass spectroscopy gas detection system or configured to perform one or more operations of multi-pass spectroscopy gas detection system.

The system operation may involve coordinated interaction between the optical path configuration, gas sample handling, thermal compensation mechanism, and sensor data collection to achieve reliable gas detection and analysis. The optical path through the thermally compensated multi-pass spectroscopy gas cell 100 may begin with light entering through one of the fused silica wedged windows (e.g., light inlet window 113) positioned at an end of the gas cell assembly. The wedged geometry of the light inlet window 113 may direct the incident light beam into the gas cell volume while minimizing unwanted reflections that could interfere with spectroscopic measurements. The light beam may travel through the gas-filled volume defined by the PVDF inner tube 103 within the carbon steel cylinder (e.g., carbon steel cell housing 102), where the beam may interact with gas molecules present in the sample.

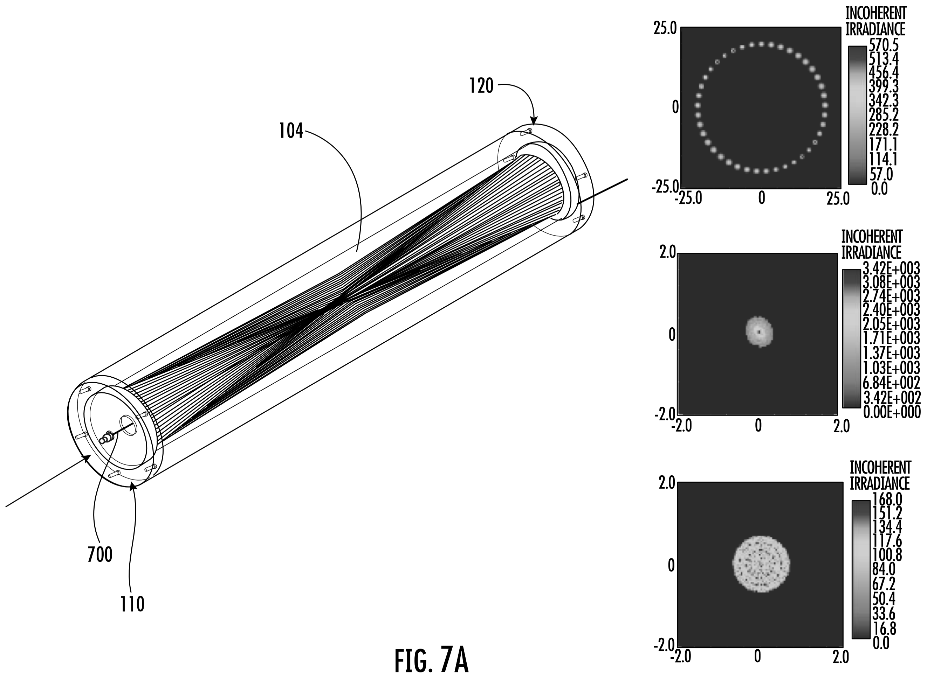

For example, as shown in FIG. 7A, light beam 700 from an emitter, such as one or more optical frequency combs, may pass through the light inlet through-hole 112 of the first mirror 110 and through the light inlet window 113 into the chamber 104. The inner surfaces of the mirrors 110, 120 may be shaped and positioned such that the light beam 700 is reflected back and forth between the mirrors 110, 120 according to a pattern that avoids collisions between any of the reflections. This multi-pass configuration may allow the effective optical path length to be many times longer than the physical length of the cell. The multi-pass optical configuration may be achieved through multiple reflections between the pair of mirrors positioned at opposite ends of the gas cell assembly. The mirrors may be mounted in the PVDF mirror holders and separated by the nominal distance (e.g., 385.2 mm in some examples), creating an optical cavity (e.g., chamber 104) that enables the light beam to traverse the gas sample multiple times before exiting the system. Each reflection between the mirrors may increase the effective optical path length through the gas sample, thereby enhancing the absorption signal strength for improved detection sensitivity.

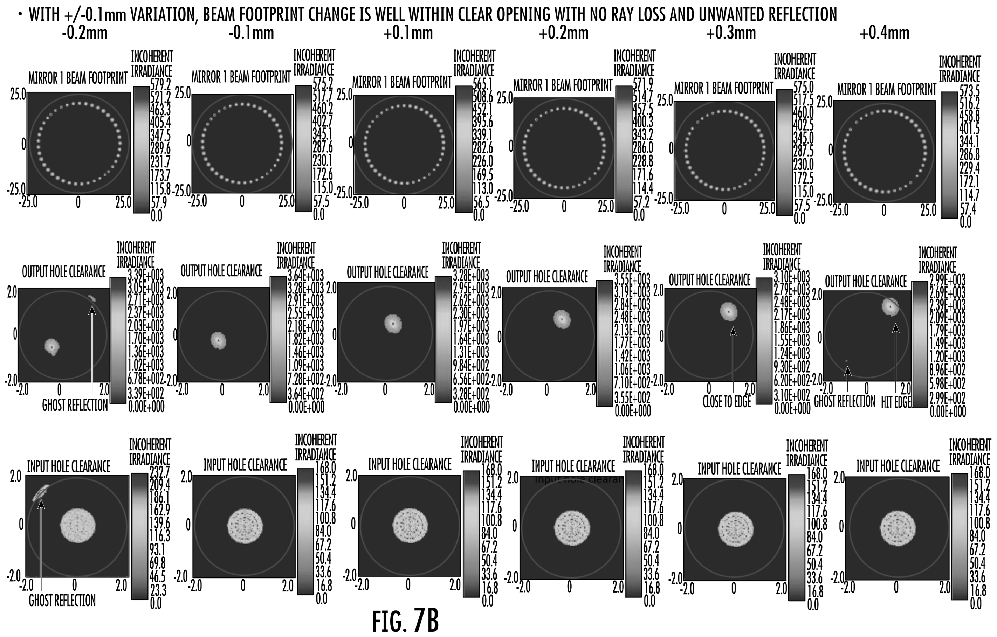

The light beam may undergo numerous reflections between the mirrors, with each pass through the gas sample contributing to the cumulative absorption signal. The multi-pass configuration may multiply the effective path length by the number of reflections, enabling detection of trace gas concentrations that might not be measurable with a single-pass optical system. As shown, in FIG. 7B, the optical path may be designed to ensure that the beam footprint remains within the clear apertures of the mirrors and windows throughout the reflection sequence.

In various embodiments, after reflecting back and forth according to the pattern determined by the surfaces of the mirrors (which may involve dozens or hundreds of reflections), for example, the light beam 700 may exit the chamber 104 through the light outlet window 123) and through the light outlet through-hole 122 defined in second mirror 120 to be received by a light receiver. For example, after completing the multi-pass sequence, the light beam may exit the gas cell through the light outlet window 123 and through light outlet through-hole 122 at the opposite end of the gas cell relative to the light inlet window 113 and light inlet through-hole 112. The wedged geometry of the exit window may prevent reflected light from re-entering the optical system while providing efficient transmission of the analyzed light beam to external detection equipment. The transmitted light may carry spectroscopic information about the gas sample composition based on wavelength-specific absorption that occurred during the multi-pass propagation.

Gas flow through the cell may occur simultaneously with optical measurements, enabling continuous monitoring or batch analysis depending on the application requirements. The gas inlet port 106 may introduce fresh gas samples into the PVDF inner tube volume, while the gas outlet port 108 may remove analyzed gases from the gas cell. The gas flow path may be designed to ensure uniform gas distribution throughout the optical path region while maintaining stable flow conditions that do not introduce optical disturbances.

The gas flow configuration may enable the system to analyze flowing gas streams in real-time applications or to process discrete gas samples in batch analysis modes. During continuous monitoring, the gas composition within the optical path may change as new gas enters through the inlet port and analyzed gas exits through the outlet port. The flow rate and residence time may be controlled to ensure adequate measurement time while maintaining representative sampling of the gas stream being monitored.

The thermal compensation mechanism may maintain stable mirror alignment and separation as temperature varies across the operating range from -20°C to +60°C. As ambient temperature changes, the carbon steel cylinder may expand or contract according to its coefficient of thermal expansion in the range of 11.2 to 12.0 × 10⁻⁶ per degree Celsius. Simultaneously, the PVDF mirror holders may expand or contract at a much higher rate according to their coefficient of thermal expansion in the range of 102 to 158 × 10⁻⁶ per degree Celsius.

The thermal compensation may function through the geometric relationship where the thermal expansion of the carbon steel cylinder length may be balanced against twice the thermal expansion of the mirror holder lengths. As temperature increases, the carbon steel cylinder may expand and tend to increase the mirror separation distance. At the same time, the PVDF mirror holders may expand at approximately ten times the rate of the carbon steel, extending further into the cylinder and tending to decrease the mirror separation distance by an amount that substantially cancels the cylinder expansion effect.

The non-linear temperature dependent characteristics of PVDF may provide additional thermal compensation benefits that extend the operational temperature range beyond what would be achievable with linear thermal expansion alone. At low and high temperatures, the combined slightly positive coefficient of thermal expansion may exhibit positive values, causing slight increases in mirror separation. At mid temperatures, the combined effective coefficient may exhibit slightly negative values, causing slight decreases in mirror separation as temperature. This bi-directional behavior may enable the system to maintain mirror separation within the ±0.1 mm tolerance across the full -20°C to +60°C temperature range.

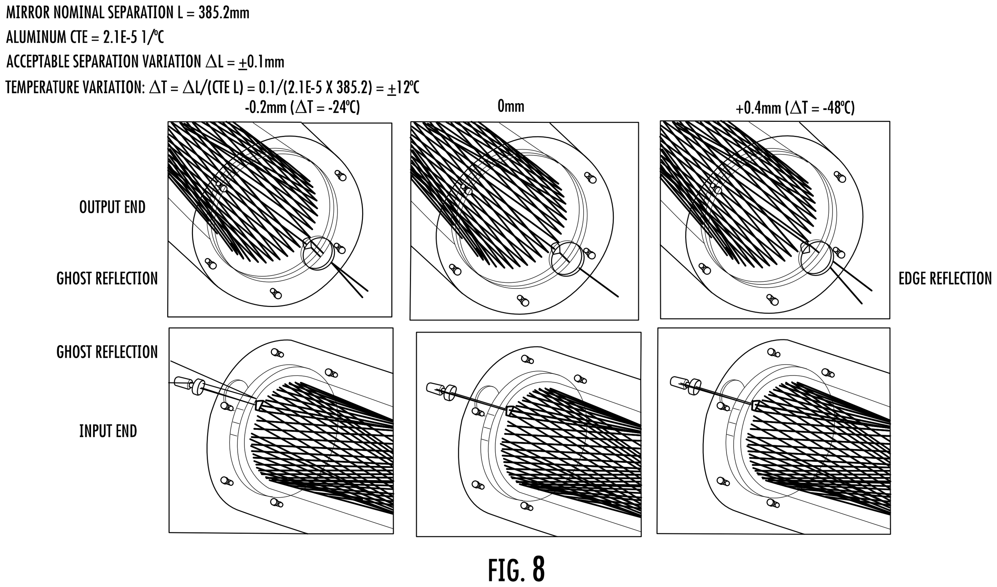

As shown in FIG. 8, the thermal compensation may maintain optical performance even when mirror separation variations reach ±0.4 mm, providing operational margin beyond the nominal ±0.1 mm specification. The optical system design may accommodate these larger separation variations without experiencing ray loss or unwanted reflections that would degrade measurement quality. This tolerance margin may ensure robust operation under extreme conditions or manufacturing variations that exceed the nominal design parameters.

The combined pressure-temperature sensor may collect real-time data about gas conditions within the cell during spectroscopic measurements. The pressure measurements may indicate the gas density within the optical path, which may affect the strength of absorption signals and the relationship between absorption measurements and gas concentration. The temperature measurements may provide information about gas temperature effects on molecular absorption characteristics and may enable temperature-corrected analysis of spectroscopic data.

The pressure and temperature data may be used in conjunction with optical absorption measurements to improve the accuracy of gas concentration determinations. Pressure corrections may account for the effects of gas density variations on absorption line strengths, while temperature corrections may account for temperature-dependent changes in absorption cross-sections and line broadening effects. The combined sensor data may enable real-time compensation for pressure and temperature effects that could otherwise introduce errors in gas concentration calculations.

The sensor data may also provide system monitoring capabilities that enable detection of operational issues or changes in gas flow conditions. Pressure readings may indicate proper gas flow through the inlet and outlet ports and may detect blockages, leaks, or other flow-related problems. Temperature readings may provide feedback about the thermal compensation performance and may enable verification that the system is operating within the designed temperature range where thermal compensation is effective.

The integration of optical measurements with pressure and temperature data may enable advanced analysis techniques that improve measurement reliability and accuracy. The coordinated data collection may support algorithms that account for the complex interactions between gas properties, optical absorption characteristics, and environmental conditions. This integrated approach may provide more robust gas detection and analysis capabilities compared to systems that rely solely on optical measurements without environmental compensation.

The sealing system comprising seal rings and fasteners may maintain gas containment throughout the operational temperature range while allowing the thermal compensation mechanism to function properly. The seals may accommodate the differential thermal expansion between carbon steel and PVDF components without losing sealing effectiveness or constraining the thermal movements needed for compensation. The sealed assembly may prevent gas leakage that could affect measurement accuracy while enabling the coordinated operation of optical, thermal, and gas flow components.

In this regard, by, utilizing PVDF material for various components of the thermally compensated multi-pass spectroscopy gas cell 100, as described above, example embodiments of the present disclosure provide a gas cell 100 with properties that are desirable in multi-pass gas cell applications. These properties include, but are not limited to, high structural stability, improved chemical resistance (particularly important for corrosive gases), and low cost (e.g., low material cost and low manufacturing cost compared to traditional low-expansion materials like Invar or Zerodur). In various embodiments, the low CTE carbon steel gas cell housing 102 with the high CTE PVDF tube 103 components (including tube 103 and mirror holders 111, 121) form a stable gas cell 100. In various embodiments, the high CTE PVDF mirror holders 111, 121 provide compensation against carbon steel cell cylinder housing 102, which also houses the inlet optical window element 113 and outlet optical window element 123 (e.g., by housing the PVDF window holders 116, 126 comprising the windows 113, 123).

### Example Thermally Compensated Multi-Pass Spectroscopy Gas Detection System

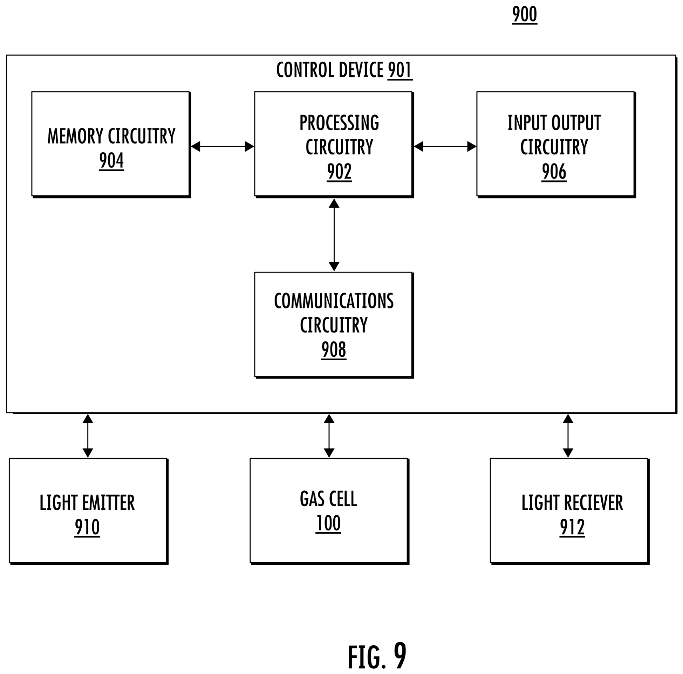

Referring now to FIG. 9, a block diagram of an example thermally compensated multi-pass spectroscopy gas detection system 900 in accordance with example embodiments of the present disclosure is provided. The example system 900 may include a thermally compensated multi-pass spectroscopy gas cell such as the example thermally compensated multi-pass spectroscopy gas cell, a control device 901, light emitter 910, and/or light receiver 912. The light emitter 910 may be configured to emit light towards and/or into the gas cell 100, as described above. The light receiver 912 may be configured to receive light that exits the gas cell 100, as described above.

The example control device 901 show in FIG. 9 comprises a processor or processing circuitry 902, memory circuitry 904, input/output circuitry 906, and communications circuitry 908. In some embodiments, one or more portions of the control device 901 (e.g., one or more of the components thereof) are configured to execute and perform the operations described herein. In some embodiments, the control device 901 may be configured to control operation of the light emitter 910, the light receiver 912, and/or the gas cell 100. For example, in such some embodiments, the control device 901 may function as a controller within the system 900. In some embodiments,, the control device 901 may be configured to analyze the output from the gas cell 100 or received by the light receiver 912 to determine the presence of one or more target gases.

Although components are described with respect to functional limitations, it should be understood that at least some of the particular implementations necessarily include the use of particular computing hardware. It should also be understood that in some embodiments certain of the components described herein include similar or common hardware. For example, in some embodiments two sets of circuitries both leverage use of the same processor(s), memory(ies), circuitry(ies), and/or the like to perform their associated functions such that duplicate hardware is not required for each set of circuitries.

Processing circuitry 902 may be embodied in a number of different ways. In various embodiments, the use of the terms processor or processing circuitry should be understood to include a single core processor, a multi-core processor, multiple processors internal to the example device 901, and/or one or more remote or cloud processor(s) external to the example device 901. In some example embodiments, processing circuitry 902 may include one or more processing devices configured to perform independently. Alternatively, or additionally, processing circuitry 902 may include one or more processor(s) configured in tandem via a bus to enable independent execution of operations, instructions, pipelining, and/or multithreading.

In an example embodiment, the processing circuitry 902 may be configured to execute instructions stored in the memory circuitry 904 or otherwise accessible to the processor. Alternatively, or additionally, the processing circuitry 902 may be configured to execute hard-coded functionality. As such, whether configured by hardware or software methods, or by a combination thereof, processing circuitry 902 may represent an entity (e.g., physically embodied in circuitry) capable of performing operations according to embodiments of the present disclosure while configured accordingly. Alternatively, or additionally, processing circuitry 902 may be embodied as an executor of software instructions, and the instructions may specifically configure the processing circuitry 902 to perform the various algorithms embodied in one or more operations described herein when such instructions are executed. In some embodiments, the processing circuitry 902 includes hardware, software, firmware, and/or a combination thereof that performs one or more operations described herein.

In some embodiments, the processing circuitry 902 (and/or co-processor or any other processing circuitry assisting or otherwise associated with the processor) is/are in communication with the memory circuitry 904 via a bus for passing information among components of the example device 901.

Memory or memory circuitry 904 may be non-transitory and may include, for example, one or more volatile and/or non-volatile memories. In some embodiments, the memory circuitry 904 includes or embodies an electronic storage device (e.g., a computer readable storage medium). In some embodiments, the memory circuitry 904 is configured to store information, data, content, applications, instructions, or the like, for enabling the example device 901 to carry out various operations and/or functions in accordance with example embodiments of the present disclosure.

Input/output circuitry 906 may be included in the example device 901. In some embodiments, input/output circuitry 906 may provide output to the user and/or receive input from a user. The input/output circuitry 906 may be in communication with the processing circuitry 902 to provide such functionality. The input/output circuitry 906 may comprise one or more user interface(s). In some embodiments, a user interface may include a display that comprises the interface(s) rendered as a web user interface, an application user interface, a user device, a backend system, or the like. In some embodiments, the input/output circuitry 906 also includes a keyboard, a mouse, a joystick, a touch screen, touch areas, soft keys a microphone, a speaker, or other input/output mechanisms. The processing circuitry 902 and/or input/output circuitry 906 may be configured to control one or more operations and/or functions of one or more user interface elements through computer program instructions (e.g., software and/or firmware) stored on a memory accessible to the processor (e.g., memory circuitry 904, and/or the like). In some embodiments, the input/output circuitry 906 includes or utilizes a user-facing application to provide input/output functionality to a computing device and/or other display associated with a user. In some embodiments, the input/output circuitry 906 one or more indicator lights or the like for providing a user notification (e.g., an alert or warning).

Communications circuitry 908 may be included in the example device 901. The communications circuitry 908 may include any means such as a device or circuitry embodied in either hardware or a combination of hardware and software that is configured to receive and/or transmit data from/to a network and/or any other device, circuitry, or module in communication with the example device 901. In some embodiments the communications circuitry 908 includes, for example, a network interface for enabling communications with a wired or wireless communications network. Additionally, or alternatively, the communications circuitry 908 may include one or more network interface card(s), antenna(s), bus(es), switch(es), router(s), modem(s), and supporting hardware, firmware, and/or software, or any other device suitable for enabling communications via one or more communications network(s). In some embodiments, the communications circuitry 908 may include circuitry for interacting with an antenna(s) and/or other hardware or software to cause transmission of signals via the antenna(s) and/or to handle receipt of signals received via the antenna(s). In some embodiments, the communications circuitry 908 enables transmission to and/or receipt of data from a user device, one or more sensors, and/or other external computing device(s) in communication with the example device 901.

In some embodiments, two or more of the sets of circuitries 902-908 are combinable. Alternatively, or additionally, one or more of the sets of circuitries 902-908 perform some or all of the operations and/or functionality described herein as being associated with another circuitry. In some embodiments, two or more of the sets of circuitries 902-908 are combined into a single module embodied in hardware, software, firmware, and/or a combination thereof.

While the description above provides an example system 900 and example control device 901, it is noted that the scope of the present disclosure is not limited to the description above. In some examples, an example system 900 and/or control device in accordance with the present disclosure may be in other forms. In some examples, an example system 900 and/or control device 901 may comprise one or more additional and/or alternative elements, and/or may be structured differently than that illustrated in FIG. 9.

Operations and processes described herein support combinations of means for performing the specified functions and combinations of operations for performing the specified functions. It will be understood that one or more operations, and combinations of operations, may be implemented by special purpose hardware-based computer systems which perform the specified functions, or combinations of special purpose hardware and computer instructions.

In some example embodiments, certain ones of the operations herein may be modified or further amplified as described below. Moreover, in some embodiments additional optional operations may also be included. It should be appreciated that each of the modifications, optional additions or amplifications described herein may be included with the operations herein either alone or in combination with any others among the features described herein.

The foregoing method and process descriptions are provided merely as illustrative examples and are not intended to require or imply that the steps of the various embodiments must be performed in the order presented. As will be appreciated by one of skill in the art the order of steps in the foregoing embodiments may be performed in any order. Words such as "thereafter," "then," "next," and similar words are not intended to limit the order of the steps; these words are simply used to guide the reader through the description of the methods. Further, any reference to claim elements in the singular, for example, using the articles "a," "an" or "the," is not to be construed as limiting the element to the singular and may, in some instances, be construed in the plural.

While various embodiments in accordance with the principles disclosed herein have been shown and described above, modifications thereof may be made by one skilled in the art without departing from the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above, but is defined by the claims which follow, that scope including all equivalents of the subject matter of the claims. Each and every claim is incorporated as further disclosure into the specification and the claims are embodiment(s) of the present disclosure. Furthermore, any advantages and features described above may relate to specific embodiments but shall not limit the application of such issued claims to processes and structures accomplishing any or all of the above advantages or having any or all of the above features.

In addition, the section headings used herein are provided for consistency with the suggestions under 37 C.F.R. § 1.77 or to otherwise provide organizational cues. These headings shall not limit or characterize the disclosure set out in any claims that may issue from this disclosure. For instance, a description of a technology in the "Background" is not to be construed as an admission that certain technology is prior art to any disclosure in this disclosure. Neither is the "Summary" to be considered as a limiting characterization of the disclosure set forth in issued claims. Furthermore, any reference in this disclosure to "disclosure" or "embodiment" in the singular should not be used to argue that there is only a single point of novelty in this disclosure. Multiple embodiments of the present disclosure may be set forth according to the limitations of the multiple claims issuing from this disclosure, and such claims accordingly define the disclosure, and their equivalents, which are protected thereby. In all instances, the scope of the claims shall be considered on their own merits in light of this disclosure but should not be constrained by the headings set forth herein.

Also, systems, subsystems, apparatuses, techniques, and methods described and illustrated in the various embodiments as discrete or separate may be combined or integrated with other systems, modules, techniques, or methods without departing from the scope of the present disclosure. Other devices or components shown or discussed as coupled to, or in communication with, each other may be indirectly coupled through some intermediate device or component, whether electrically, mechanically, or otherwise. Other examples of changes, substitutions, and alterations are ascertainable by one skilled in the art and could be made without departing from the scope disclosed herein.

Many modifications and other embodiments of the disclosure set forth herein will come to mind to one skilled in the art to which these embodiments pertain having the benefit of teachings presented in the foregoing descriptions and the associated figures. Although the figures only show certain components of the apparatuses and systems described herein, various other components may be used in conjunction with the components and structures disclosed herein. Therefore, it is to be understood that the disclosure is not to be limited to the specific embodiments disclosed and that modifications and other embodiments are intended to be included within the scope of the appended claims. For example, the various elements or components may be combined, rearranged, or integrated in another system or certain features may be omitted or not implemented. Moreover, the steps in any method described above may not necessarily occur in the order depicted in the accompanying drawings, and in some examples one or more of the steps depicted may occur substantially simultaneously, or additional steps may be involved. Although specific terms are employed herein, they are used in a generic and descriptive sense only and not for purposes of limitation.

## Claims

1. A thermally compensated multi-pass spectroscopy gas cell comprising:
a cylindrical cell housing constructed from a first material having a first coefficient of thermal expansion (CTE) *CTE_{cell_cyl}* and defining an interior;
a first mirror assembly comprising a first mirror holder and a first mirror secured within the first mirror holder; and
a second mirror assembly comprising a second mirror holder and a second mirror secured within the second mirror holder;
wherein the first mirror assembly and the second mirror assembly are positioned at opposite ends of the cylindrical cell housing to create a multi-pass optical configuration, and
wherein, the first mirror holder and the second mirror holder are constructed from a second material having a second CTE *CTE_{mirror_hldr}* that is greater than the first CTE to provide thermal compensation for stable mirror separation across operating temperature range.

2. The thermally compensated multi-pass spectroscopy gas cell of claim 1, wherein the cylindrical cell housing defines a first length *L_{cell_cyl}* and each of the first mirror holder and the second mirror define a second length *L_{mirror_hldr},* wherein the first length and the second length are selected based on a thermal compensation relationship L_{cell_cyl} × *CTE_{cell_cyl}*= 2 × *L_{mirror_hldr}* × *CTE_{mirror_hldr}.*

3. The thermally compensated multi-pass spectroscopy gas cell of claim 1, wherein the first material comprises carbon steel.

4. The thermally compensated multi-pass spectroscopy gas cell of claim 1, wherein the first CTE is in range of 11.2 to 12.0 × 10⁻⁶ per degree Celsius.

5. The thermally compensated multi-pass spectroscopy gas cell of claim 1, wherein the second material comprises polyvinylidene fluoride (PVDF).

6. The thermally compensated multi-pass spectroscopy gas cell of claim 1, wherein the second CTE is in a range of approximately 102 to 158 × 10⁻⁶ per degree Celsius.

7. The thermally compensated multi-pass spectroscopy gas cell of claim 1, further comprising an inner tube positioned within the interior of the cylindrical cell housing, wherein the inner tube is constructed of from the second material.

8. The thermally compensated multi-pass spectroscopy gas cell of claim 1, wherein the first mirror and the second mirror are constructed from fused silica.

9. The thermally compensated multi-pass spectroscopy gas cell of claim 1, further comprising:
a first window assembly and a second window assembly positioned at the opposite ends of the cylindrical cell housing, wherein the first window assembly comprises a first window holder and a light inlet window secured within the first window holder, and wherein the second window assembly comprises a second window holder and a light outlet window secured with the second window holder.

10. The thermally compensated multi-pass spectroscopy gas cell of claim 9, wherein the first window holder and the second window holder are constructed from the second material.

11. The thermally compensated multi-pass spectroscopy gas cell of claim 10, wherein the light inlet window and the light outlet window are constructed from fused silica and have a wedged geometry.

12. The thermally compensated multi-pass spectroscopy gas cell of claim 11, wherein the first mirror defines a light inlet through-hole and the second mirror defines a light outlet through-hole, wherein the light inlet through-hole and the light inlet window are aligned, and wherein the light outlet through-hole and the light outlet window are aligned.

13. The thermally compensated multi-pass spectroscopy gas cell of claim 1, further comprising a chamber defined at least in part by the cylindrical cell housing, the first mirror, and the second mirror.

14. The thermally compensated multi-pass spectroscopy gas cell of claim 13, further comprising a
a gas inlet port for introducing gas samples into the chamber; and
a gas outlet port for removing gas samples from the chamber.

15. A gas detection system comprising:
a thermally compensated multi-pass spectroscopy gas cell comprising:
a cylindrical cell housing constructed from a first material having a first CTE and defining an interior;
a first mirror assembly comprising a first mirror holder and a first mirror secured within the first mirror holder; and
a second mirror assembly comprising a second mirror holder and a second mirror secured within the second mirror holder; wherein the first mirror assembly and the second mirror assembly are positioned at opposite ends of the cylindrical cell housing to create a multi-pass optical configuration, and wherein, the first mirror holder and the second mirror holder are constructed from a second material having a second CTE that is greater than the first CTE to provide thermal compensation for stable mirror separation across operating temperature range;
a light emitter configured to emit light beam toward the thermally compensated multi-pass spectroscopy gas cell; and
a light receiver configured to receive light exiting the compensated multi-pass spectroscopy gas cell.
